# EUROPÄISCHE PATENTANMELDUNG

(11) **EP 0 965 579 A2**
(43) Veröffentlichungstag der Anmeldung: **22.12.1999**
(21) Anmeldenummer: 99111183.2
(22) Anmeldetag: 09.06.1999
(51) Int. Cl.: C07C 209/72, B01J 23/16, B01J 23/46

(54) **Verfahren zur Herstellung von cycloaliphatischen Aminen**

(30) Priorität: 19.06.1998 DE 19827282
(71) Anmelder: Bayer Aktiengesellschaft, 51368 Leverkusen (DE)
(72) Erfinder: Langer, Reinhard, Dr., 47918 Tönisvorst (DE); Petruck, Gerd-Michael, Dr., 40699 Erkrath (DE)

(57) **Zusammenfassung**

Die vorliegende Erfindung betrifft ein Niederdruckverfahren zur Hydrierung von aromatischen Aminen zu den entsprechenden cycloaliphatischen Aminen in Gegenwart von Rhodium-Katalysatoren die gegebenenfalls mit Ir, Ru, Os, Pd oder Pt oder Gemischen dieser Metalle modifiziert sind, auf mit Oxiden der Metalle Cr, Mo, W, Mn oder Re oder Gemischen dieser Oxide modifizierten Trägern.

## Beschreibung

Die vorliegende Erfindung betrifft ein Niederdruckverfahren zur Hydrierung von aromatischen Aminen zu den entsprechenden cycloaliphatischen Aminen in Gegenwart von Rhodium-Katalysatoren die gegebenenfalls mit einem Edelmetall der Reihe Ir, Ru, Os, Pd oder Pt oder Gemischen dieser Metalle modifiziert sind, auf mit Oxiden der Metalle Cr, Mo, W, Mn, oder Re oder Gemischen dieser Oxide modifizierten Trägern.

FR 1.530.477 beschreibt ein Niederdruckverfahren in dem Anilin an Pd-Trägerkatalysatoren bei Temperaturen zwischen 175°C und 190°C im Wasserstoffstrom mit hohen Mengen Ammoniak umgesetzt wird. Das Produkt enthält große Mengen Dicyclohexylamin.

EP-A 0 560 127 beschreibt ein Niederdruckverfahren in dem Anilin an basisch modifizierten Ru-Pd-Trägerkatalysatoren umgesetzt wird. Die Katalysatoren sind nur sehr gering belastbar, müssen in Gegenwart großer Ammoniakmengen betrieben werden und produzieren trotzdem Cyclohexylamin nur mit einer maximal 89%igen Selektivität.

US 5 360 934 offenbart ein Verfahren zur Hydrierung von aromatischen Aminen an einem Rhodiumkatalysator, der auf einen Träger aus κ-, θ- oder δ-Al₂O₃ aufgebracht ist. US 4 960 941 offenbart ebenfalls ein Verfahren zur Hydrierung von aromatischen Aminen an einem Rhodiumkatalysator. In diesem Fall ist der Rhodiumkatalysator auf einen Träger aus TiO₂ aufgebracht. In beiden Fällen wird die Hydrierung in flüssiger Phase unter Druck durchgeführt.

US 5 773 657 offenbart die Hydrierung aromatischer Verbindungen, die mindestens eine Aminogruppe am aromatischen Kern tragen. Das Verfahren wird in flüssiger Phase an einem Rutheniumkatalysator bei Drucken über 50 bar, bevorzugt in einem Druckbereich von 150 bis 300 bar, durchgeführt.

US 5 023 226 bezieht sich auf Rutheniumkatalysatoren, die zusätzlich Palladium und/oder Platin enthalten und auf einen Al₂O₃- oder Aluminiumspinellträger aufgebracht sind, der mit Chrom- und Manganverbindungen behandelt wurde.

Ein Verfahren, welches bei niederen Drucken aromatische Amine an einer stationären Katalysatorschüttung selektiv zu cycloaliphatischen Aminen umsetzt, bei dem der Katalysator standfest und mit mehr als 0,1 kg aromatischem Amin, beispielsweise Anilin, pro Liter Katalysator und Stunde belastbar ist und kein oder nur wenig Ammoniak im Kreis geführt werden muß, ist unbekannt.

Daher sind auch Anmeldungen zur Niederdruckhydrierung von aromatischen Aminen mit Katalysatoren die Rh als Edelmetallkomponente enthalten nicht bekannt obwohl in der Literatur darüber spekuliert wird, daß Rh-Katalysatoren für Niederdruckhydrierungen von Anilinen geeignet sein sollen (P.N. Rylander, Catalytic Hydrogenation over Platinum Metals, Academic Press 1967 S. 331-363; P.N. Rylander, Hydrogenation Methods, Academic Press 1985, S. 123-133).

Ein Vorurteil gegen die Entwicklung eines Rh-Katalysators zur Produktion von Cyclohexylaminen bei niederem Druck erzeugte eine vor wenigen Jahren veröffentlichte Arbeit zur Gasphasen-Hydrierung von Anilin an Rh auf γ-Al₂O₃: Bei 1 atm und 200°C wurden mit steigendem Rh-Gehalt des Katalysators zwar steigende Umsätze erzielt, jedoch war die Cyclohexylamin-Selektitivität mit ca. 20% unabhängig vom Umsatz sehr gering. Die Dicyclohexylaminselektivität sank sogar mit steigendem Rh-Gehalt und damit mit steigendem Umsatz von 40 auf 20%, so daß zum größten Teil unerwünschte Produkte erhalten wurden. (V. Vishwanathan, S. Narayanan, J. Chem. Soc., Chem. Commun., 1990, 78-80).

Die Veröffentlichung legt nahe, daß Rhodium-Katalysatoren für technische Hydrierungen von Anilinen zu Cyclohexylaminen bei niederen Drucken in der Gasphase ungeeignet sind.

Der vorliegenden Erfindung lag die Aufgabe zugrunde, ein selektives und belastbares Niederdruckverfahren zur Hydrierung von aromatischen Aminen, beispielsweise Anilinen, zu cycloaliphatischen Aminen (beispielsweise Cyclohexylaminen) zu finden.

Überraschenderweise wurde gefunden, daß Kontakte die Rh auf speziell behandelten Trägern enthalten potente Katalysatoren bei der Realisierung eines Verfahrens zur Niederdruckhydrierung von aromatischen Aminen sind.

Gegenstand der Erfindung ist ein Verfahren zur Hydrierung von aromatischen Aminen zu cycloaliphatischen Aminen bei Drucken zwischen 0,5 und 40 bar an mit Basen behandelten Edelmetallträgerkatalysatoren, dadurch gekennzeichnet, daß der Träger mit Salzen von Cr, Mo, W, Mn oder Re oder Gemischen dieser Salze belegt worden ist, und daß der so erhaltene Träger mit Rh als Edelmetall und gegebenenfalls Ir; Ru, Os, Pd und/oder Pt als zusätzlicher Edelmetallkomponente aktiviert worden ist.

Geeignete Ausgangsverbindungen für das erfindungsgemäße Verfahren sind aromatische Amine wie sie beispielsweise in DE-AS 2 502 894 und US 3 636 108 beschrieben sind. Bevorzugt sind Anilin, C₁-C₆-Alkylaniline sowohl im Kern wie am Stickstoff alkyliert, C₁-C₆-alkylierte Diaminobenzole, Aminonaphthaline und C₁-C₃-alkylierte Aminonaphthaline, Diaminonaphthaline und Diamino-diphenyl-C₁-C₃-alkane.

Genannt seien beispielsweise Anilin, N-Methylanilin, N-Ethylanilin, N,N-Dimethylanilin, N,N-Diethylanilin, N-Ethyltoluidin, N-Cyclohexylanilin, N-Cyclohexylidenanilin, o-, m-, p-Toluidin, 2,4-, 2,6-, 2,3-Diamino-toluol, Diphenylamin, 1- und 2- Aminonaphthalin, 1,4-, 1,5-, 2,5-, 2,6-, 2,7-Diaminonaphthalin und die isomeren Diaminophenylmethane.

Bevorzugt genannt seien beispielsweise Anilin, N-Methylanilin, N-Ethylanilin, N,N-Dimethylanilin, N,N-Diethylanilin, N-Cyclohexylanilin, N-Cyclohexylidenanilin, o-, m-, p-Toluidin, 2,4-, 2,6-, 2,3-Diamino-toluol, Diphenylamin.

Besonders bevorzugt genannt seien beispielsweise Anilin, 2,4- und 2,6-Diaminotoluol.

Ganz besonders bevorzugt wird das erfindungsgemäße Verfahren eingesetzt um Anilin zu hydrieren.

Die Edelmetallträgerkatalysatoren für das erfindungsgemäße Verfahren bestehen aus einem Träger, der mit einem Salz der Metalle Cr, Mo, W, Mn oder Re oder Gemischen dieser Salze belegt worden ist. Ferner beinhalten die Edelmetallträgerkatalysatoren Rh als Edelmetall und gegebenenfalls eine zusätzliche Edelmetallkomponente der Reihe Ir, Ru, Os, Pd und/oder Pt.

Träger für die Edelmetallträgerkatalysatoren des erfindungsgemäßen Verfahrens sind Tonerden, Al₂O₃ in den verschiedenen Modifikationen (α, κ, η, γ), weiter ansonsten für Edelmetalle übliche Träger wie TiO₂, Kieselgur, Silicagel, BaCO₃, CaCO₃, ZnO, MgO, Bims, ZrO₂, Aktivkohle und natürlich die Oxide bzw. Oxidhydrate von Cr, Mo, W, Mn und/oder Re. Bevorzugte Träger sind TiO₂, BaCO₃, MgO, besonders bevorzugt γ-Al₂O₃ oder die Oxide bzw. Oxidhydrate von Cr, Mo, W, Mn und/oder Re, ganz besonders bevorzugt γ- Al₂O₃.

Der Träger kann als Pulver oder in stückiger Form als Kugeln oder als Extrudat wie Ringe, Wagenräder u.s.w. eingesetzt werden, ferner sind Formkörper wie z.B. Wabenkörper oder Kreuzkanalstrukturen verwendbar.

Vorzugsweise wird ein Träger mit großer BET-Oberfläche eingesetzt. Die BET-Oberfläche sollte oberhalb 50 m²/g, bevorzugt zwischen 100 und 500 m²/g, besonders bevorzugt zwischen 200 und 400 m²/g liegen.

Enthält der Träger Oxide oder Oxidhydrate von Metallen der Reihe Cr, Mo, W, Mn, oder Re oder Gemische solcher Oxide oder Oxidhydrate kann gegebenenfalls auf die nachfolgend beschriebene Modifikation des Trägers vor dem Aufbringen der Edelmetallkomponenten verzichtet werden.

Wird ein Cr, Mo, W, Mn oder Re-freier Träger eingesetzt, ist jener zuerst mit einer oder meherer dieser Komponenten zu belegen. Dies kann z.B. durch Tränken oder Besprühen des Trägers mit geeigneten Salzen dieser Elemente geschehen. Durch Trocknen und danach Tempern bei Temperaturen von ca. 200 bis 450°C werden die aufgebrachten Salze in aufdem Träger haftende Verbindungen überführt. Das Aufbringen der Verbindungen von Cr, Mo, W, Mn, und/oder Re kann jedoch auch durch gemeinsames Ausfällen von Oxid-Hydroxid-Gemischen aufdem getränkten Träger mit Alkali-, Erdalkali-, oder Ammonium-Hydroxiden und gegebenenfalls anschließendes Auswaschen löslicher Anteile mit Wasser geschehen.

Besonders bevorzugt wird eine gleichmäßige Fällung durch langsame Freisetzung der Base durch Hydrolyse einer weniger basischen Vorstufe, besonders geeignet sind dazu Harnstoffe und Urethane, ganz besonders geeignet ist Harnstoff.

Der so vorbehandelte Träger wird getrocknet und dann beispielsweise zwischen 10 Minuten und 10 Stunden auf 200 bis 450°C, bevorzugt 250 bis 430°C erhitzt, wobei die Temperatur innerhalb dieses Bereiches auch nach und nach erhöht werden kann.

Geeignete Salze von Cr, Mo, W, Mn und Re sind beispielsweise die Acetate, Nitrate, Halogenide oder Sulfate. Ebenso geeignet sind die wasserlöslichen Oxide der höheren Oxidationsstufen, besonders die Ammoniumsalze der Cr, Mo, W, Mn und Re -Oxide.

Bevorzugt werden Träger eingesetzt, die mit Cr- und Mn- Salzen vorbehandelt worden sind.

Nach dem gegebenenfalls durchgeführten Auswaschen löslicher Verbindungen und der Trocknung und Temperung des mit Cr, Mo, W, Mn und/oder Re modifizierten Trägers ist der Träger zur Aufnahme der übrigen Wirkstoffe bereit.

Die übrigen Wirkstoffe sind Rh und gegebenenfalls ein Edelmetall der Reihe Ir, Ru, Os, Pd, und/oder Pt, Alkali- oder Erdalkali-Hydroxid und gegebenenfalls Alkali- oder Erdalkalisulfat. Die Edelmetalle werden in Form von Lösungen ihrer Salze z.B. in Wasser aufgebracht. Als Salze sind z.B. geeignet die Halogenide, bevorzugt die Chloride, Acetate, Nitrate und Acetylacetonate. Als Alkalihydroxid ist z.B. NaOH oder KOH geeignet, als Erdalkalihydroxid z.B. Mg(OH)₂.

Als Sulfatkomponente ist z.B. K₂SO₄ zu nennen. Die Verbindungen können einzeln oder zusammen durch tränken oder besprühen aufgebracht werden. Zwischen jedem Tränkungsschritt erfolgt eine Trocknung.

Das Alkali- oder Erdalkali-Hydroxid kann vor oder nach den Edelmetallen aufgebracht werden.

Bevorzugt wird zuerst Rh, dann gegebenenfalls die Edelmetalle zur Modifizierung aufgebracht, gefolgt vom Alkalihydroxid und gegebenenfalls vom Alkalisulfat und gegebenenfalls einer weiteren Tränkung mit Base.

Bevorzugt wird nach jeder Tränkung mit Edelmetall gegebenenfalls mit z.B. Wasserstoff oder einem anderen Reduktionsmittel reduziert, in jedem Fall wird am Ende der letzten Trocknung z.B. mit Wasserstoff bei Temperaturen zwischen 80 und 350°C reduziert.

Der fertige Edelmetallträgerkatalysator enthält zwischen 0,1 und 10 Gew.-%, bevorzugt zwischen 0,3 und 3 Gew.-% Edelmetall, das zwischen 100 und 30%, bevorzugt zwischen 100 und 70% aus Rh besteht, das restliche Edelmetall besteht aus Ir, Ru, Os, Pd und/oder Pt. Ferner enthält der Katalysator zwischen 0,05 und 5 Gew.-% Cr, Mo, W, Mn und/oder Re, bevorzugt Cr und Mn, weiterhin zwischen 0,05 und 15 Gew.-% Alkali- oder Erdalkalimetallionen und gegebenenfalls zwischen 0,05 und 3 Gew.-% Schwefel in Form von Verbindungen.

Bevorzugt wird im erfindungsgemäßen Verfahren ein geeigneter Edelmetallträgerkatalysator stückig in Form fester Schüttungen eingesetzt. Die Schüttungen können adiabat sein oder durch Verwendung von mit Wärmeträgern durchströmten oder umspülten Rohrbündeln thermostatisiert werden. Ebenso ist eine Kombination aus thermostatisieden und adiabaten Schüttungen vorteilhaft, oder eine Folge von adiabaten Reaktoren mit zwischen geschalteten Kühlern. Die Ausgestaltung geeigneter Reaktoren für solche Schüttungen ist Stand der Technik und dem Fachmann bekannt.

Die Umsetzung kann in der Form erfolgen, daß aromatisches Amin und Wasserstoff gegebenenfalls zusammen mit zu recyclisierenden Verbindungen, wie z.B. Wasserstoff, Ammoniak, Dicyclohexylamin erwärmt werden, die erwärmte Mischung über den Kontakt gefahren wird, ein Teil der kondensierbaren Verbindungen durch Abkühlen niedergeschlagen und zusammen mit gegebenenfalls vorher schon vorhandener Flüssigkeit ausgeschleust wird, vom verbleibenden Gasstrom ein Teil zur Ausschleusung von inerten Verbindungen abgezweigt und der Rest durch Verdichtung der Reaktion wieder zugeführt wird. Dem Reaktor wird ein gasförmiges Eduktgemisch zugeführt.

Das erfindungsgemäße Verfahren wird bei Temperaturen zwischen 50°C und 300°C, bevorzugt zwischen 100°C und 250°C, besonders bevorzugt zwischen 140°C und 200°C durchgeführt.

Die Umsetzung erfolgt in einem Druckbereich zwischen 0,5 und 40 bar, bevorzugt zwischen 0,7 und 15 bar, besonders bevorzugt zwischen 0,9 und 8 bar.

Das umzusetzende aromatische Amin kann zusammen mit Wasserstoff im molaren Verhältnis zwischen 1:500 und 1:5, bevorzugt zwischen 1:200 und 1:10, besonders bevorzugt zwischen 1:150 und 1:40 umgesetzt werden.

Zusammen mit den aromatischen Aminen und dem Wasserstoff können geringe Mengen Ammoniak über den Kontakt gefahren werden. Ammoniak verringert die Reaktionsgeschwindigkeit deutlich und verbessert die Cyclohexylamin-Selektivität nur relativ geringfügig.

Die Belastung der Kontakte im erfindungsgemäßen Verfahren kann zwischen 0,05 und 5 kg, bevorzugt zwischen 0,2 und 2 kg aromatischem Amin pro Liter Katalysator und Stunde liegen.

Bei Einsatz von Anilin liegen die Selektivitäten bezüglich Cyclohexylamin im erfindungsgemäßen Verfahren deutlich über 97 %, im allgemeinen über 99 %.

Das erfindungsgemäße Verfahren ermöglicht aromatische Amine, insbesondere Aniline in wenig aufwendigen Niederdruckapparaturen mit hoher Selektivität in cycloaliphatische Amine, insbesondere in die Cyclohexylamine umzuwandeln.

### Beispiele

### Beispiel 1 (Katalysatorpräparation)

1 L γ-Al₂O₃ der Firma Rhone-Poulenc (SPH 501, Kugeln, ⌀=4-6 mm, BET-Oberfläche ca. 350 m²/g) wurden mit 320 ml einer Lösung aus 30,1 g MnSO₄ × H₂O, 22,3 g (NH₄)₂Cr₂O₇ und 164 g Harnstoff getränkt. Der getränkte Träger wurde 1 h bei 90°C in einer gesättigten Wasserdampfatmosphäre bewegt. Danach erfolgten zwei Wäschen mit je 160 ml Wasser zur Entfernung löslicher Verbindungen. Der so erhaltene Träger wurde getrocknet und anschließend 30 Minuten bei 300°C in einer Drehtrommel getempert.
20,3 g RhCl₃ in 360 ml Wasser wurden durch Tränkung aufgebracht und der Katalysatorvorläufer anschließend bei 110°C getrocknet.
Danach wurden 320 ml einer Lösung aus 24 g NaOH und 24 g K₂SO₄ in Wasser aufgebracht, getrocknet und nochmals mit 50 g NaOH in 320 ml Wasser getränkt. Der Kontakt wurde getrocknet und 3,5 h bei 160°C im Wasserstoffstrom aktiviert. Der fertige Kontakt enthält 8 g Rh, 9,2 g Cr, 9,8 g Mn, 74 g NaOH und 24 g K₂SO₄ pro Liter.

### Beispiel 2 (Einfluß der Temperatur bei Normaldruck)

In ein ölthermostatisiertes Reaktorrohr mit einem Innendurchmesser von 2,5 cm wurden 120 ml Katalysator aus Beispiel 1 gefüllt. Die Länge der Schüttung betrug ca. 24 cm. Der Kontakt wurde 3,5 h bei 160°C im Wasserstoffstrom aktiviert und dann die in Tabelle 1 angegebenen Reaktionsbedingungen nacheinander eingestellt und gehalten.

**Tabelle 1**

| **Variationen der Reaktionstemp.: Belastung: 0,2-0,3 kg/Lxh, H**_{**2**}**/ANI = 80/1 mol/mol.** **Kat. 120 ml Kontakt aus Beispiel 1.** | | | | | | | |
|---|---|---|---|---|---|---|---|
| **Temp. °C** | **ANI %** | **Bz %** | **CA %** | **CHA %** | **DCA %** | **UK %** | **Selekt. %** |
| 220 | 25 | 1,7 | 0,1 | 70 | 0,3 | 1,7 | 93,3 |
| 200 | 4 | 1,8 | 0,1 | 91 | 1,9 | 1,8 | 94,8 |
| 180 | 1,3 | 0,4 | 0 | 96 | 1,8 | 0,4 | 97,3 |
| 160 | 0,2 | 0 | 0 | 97 | 2,6 | 0 | 97,2 |

Tabelle 1 zeigt die Zusammensetzung der Flüssigenphase nach dem Abkühlen des Produktstromes auf ca. 10°C. Man erkennt, daß der erzielte Umsatz bei Normaldruck im untersuchten Temperaturbereich mit fallender Temperatur deutlich steigt. Die erreichten Umsätze werden offensichtlich von thermodynamischen Gegebenheiten bestimmt. ANI = Anilin; Bz = Benzol; CA = Cyclohexan, CHA = Cyclohexylamin, DCA = Dicyclohexylamin, UK = unbekannte Komponenten, Selekt. = Selektivität.

### Beispiel 3 (Einfluß des Wasserstoffüberschusses bei Normaldruck)

Im Versuchsaufbau gemäß Beispiel 2 wurde nun das Wasserstoff-Anilin-Verhältnis im Eduktstrom variiert. Tabelle 2 zeigt wieder die Zusammensetzung der kondensierten Phase. Man erkennt, daß die Selektivität bezüglich Cyclohexylamin mit fallender Wasserstoffmenge leicht sinkt.

**Tabelle 2**

| **Anilin-Hydrierung, 164°C Öltemperatur, 1 atm, H**_{**2**}**-Strom.** **Kat.: 120 ml Kontakt aus Beispiel 1.** | | | | | | | |
|---|---|---|---|---|---|---|---|
| **H**_{**2**}**/Anilin mol/mol** | **Belastung kg/L×h** | **ANI %** | **CHA %** | **DCA %** | **Anon+Anol %** | **LS %** | **UK %** |
| **40** | **0,22** | **0** | **97,8** | **1,9** | **0,24** | **0,06** | **0,03** |
| **40** | **0,22** | **0** | **98,3** | **1,5** | **0,13** | **0,09** | **0,02** |
| **22** | **0,22** | **0** | **96,8** | **2,8** | **0,22** | **0,21** | **0,08** |
| **10** | **0,22** | **0** | **94,6** | **4,6** | **0,33** | **0,35** | **0,05** |
| **40** | **0,22** | **0** | **98,3** | **1,4** | **0,24** | **0,08** | **0,06** |
| **LS =** Leichtsieder, beispielsweise Verbindungen, die bei der Analyse im Gaschromatographen vor Cyclohexylamin auftreten **Anon + Anol =** Cyclohexanon + Cyclohexanol | | | | | | | |

### Beispiel 4 (Einfluß der Belastung unter Normaldruck)

**Tabelle 3**

| **Anilin-Hydrierung,164°C Öltemperatur,1 atm,H**_{**2**}**-Strom.** **Kat.: 120 ml Kontakt aus Beispiel 1.** | | | | | | | |
|---|---|---|---|---|---|---|---|
| **H**_{**2**}**/Anilin mol/mol** | **Belastung kg/L×h** | **ANI %** | **CHA %** | **DCA %** | **Anon+Anol %** | **LS %** | **UK %** |
| **22** | **0,22** | **0** | **96,8** | **2,8** | **0,22** | **0,21** | **0,08** |
| **10** | **0,22** | **0** | **94,6** | **4,6** | **0,33** | **0,35** | **0,05** |
| **40** | **0,22** | **0** | **98,3** | **1,4** | **0,24** | **0,08** | **0,06** |
| **20** | **0,44** | **2,1** | **96,0** | **1,6** | **0,11** | **0,19** | **0,03** |
| **11** | **0,44** | **4,2** | **92,7** | **2,7** | **0,08** | **0,27** | **0,05** |
| **20** | **0,44** | **3,5** | **94,5** | **1,7** | **0,14** | **0,16** | **0,05** |

Tabelle 3 zeigt den Einfluß der Belastung auf Umsatz und Selektivität. Verdoppelt man die Belastung bei Normaldruck von 0,22 auf 0,44 kg/Lxh so wird zwar das Anilin nicht mehr ganz vollständig umgesetzt, es steigt aber die Selektivität leicht an.

### Beispiel 5 (Standzeitversuch)

Tabelle 4 zeigt, daß der Katalysator aus Beispiel 1 und 2 bei Normaldruck über lange Zeiträume mit hohem Umsatz und hoher Selektivität Anilin zu Cyclohexylamin hydriert.

**Tabelle 4**

| **Anilin-Hydrierung, 164°C Öltemperatur, 1 atm, H**_{**2**}**-Strom.** **Kat.: 120 ml Kontakt aus Beispiel 1.** | | | | | | | | |
|---|---|---|---|---|---|---|---|---|
| **H**_{**2**}**/Anilin mol/mol** | **Belastung kg/Lxh** | **ANI %** | **CHA %** | **DCA %** | **Anon + Anol %** | **LS %** | **UK %** | **Standzeit h** |
| 40 | 0,22 | 0 | 97,8 | 1,9 | 0,24 | 0,06 | 0,03 | 18 |
| 40 | 0,22 | 0 | 98,3 | 1,5 | 0,13 | 0,09 | 0,02 | 133 |
| 40 | 0,22 | 0 | 98,3 | 1,4 | 0,24 | 0,08 | 0,06 | 314 |
| 40 | 0,22 | 0,17 | 98,1 | 1,5 | 0,12 | 0,06 | 0,03 | 475 |
| 40 | 0,22 | 0,17 | 97,2 | 2,0 | 0,47 | 0,05 | 0,02 | 1000 |
| 40 | 0,22 | 0,75 | 97,0 | 2,3 | 0,09 | 0 | 0,01 | 1583 |
| 43 | 0,20 | 0,95 | 96,6 | 2,4 | 0 | 0 | 0 | 2113 |

Der Katalysator ist für eine technische Cyclohexylaminproduktion besonders geeignet, weil er ferner durch Abbrennen und Reduzieren mit Wasserstoff regenerierbar ist und nochmaliges Tränken des Katalysators mit verdünnter NaOH die Selektivität für Cyclohexylamin auf über 99% erhöht!

### Beispiel 6 (Einfluß von Ammoniak unter Normaldruckdosierung)

Mischt man zum Eduktstrom im Versuch aus Beispiel 5 fünf mole Ammoniak pro mol Anilin zu, so geht der Umsatz von 100% rasch auf ca. 80% zurück. Die Selektivität war in diesem Versuch generell höher und stieg durch die Ammoniakzugabe von ca. 99,4% auf ca. 99,7%.

### Beispiel 7 (Einfluß des Wasserstoffdruckes)

Der Versuch aus Beispiel 5 wurde in einem druckfesten Reaktorrohr wiederholt, wobei nun sowohl der Druck als auch die Belastung schrittweise heraufgesetzt wurden.

**Tabelle 5**

| **Anilin-Hydrierung, 164°C Öltemperatur, H**_{**2**}**/Anilin = 40/1.** **Kat.: 120 ml Kontakt aus Beispiel 1.** | | | | | | | | |
|---|---|---|---|---|---|---|---|---|
| **Druck bar** | **Belastung kg/Lxh** | **ANI %** | **CHA %** | **DCA %** | **Anon + Anol** | **UK %** | **Selek. %** | **Standzeit h** **%** |
| 2 | 0,22 | 0 | 98,7 | 1,2 | 0 | 0,10 | 98,7 | 70 |
| 2 | 0,41 | 0 | 99,0 | 0,9 | 0,09 | 0,01 | 99,0 | 242 |
| 2 | 1,12 | 2,0 | 96,9 | 0,17 | 0,17 | 0 | 98,9 | 552 |
| 4 | 1,74 | 0 | 98,5 | 1,3 | 0,17 | 0,03 | 98,5 | 649 |
| 4 | 1,74 | 1,17 | 97,8 | 0,9 | 0,10 | 0,03 | 99,0 | 816 |
| 6 | 1,74 | 0 | 98,5 | 1,3 | 0,14 | 0,01 | 98,5 | 864 |
| 6 | 1,74 | 0,62 | 98,0 | 1,2 | 0,13 | 0,05 | 98,6 | 1200 |
| 6 | 1,74 | 0,46 | 98,4 | 1,1 | 0,12 | 0 | 98,9 | 1530 |

Man erkennt, daß durch Anwendung geringer Drucke die Belastbarkeit des Katalysators deutlich angehoben werden kann, ohne der Standzeit des Katalysators oder der Selektivität des Verfahrens zu schaden.

## Patentansprüche

1. Verfahren zur Hydrierung von aromatischen Aminen zu cycloaliphatischen Aminen bei Drucken zwischen 0,5 und 40 bar an mit Basen behandelten Edelmetallträgerkatalysatoren, dadurch gekennzeichnet, daß der Träger der Edelmetallträgerkatalysatoren mit Salzen von Metallen der Reihe Cr, Mo, W, Mn oder Re oder Gemischen solcher Salze belegt worden ist, und daß der so erhaltene Träger mit Rh als Edelmetallkomponente aktiviert wird.

2. Verfahren gemäß Anspruch 1, dadurch gekennzeichnet, daß zusätzlich zur Aktivierung mit Rh der Träger mit einem Edelmetall der Reihe Ir, Ru, Os, Pd und/oder Pt aktiviert wird.

3. Verfahren gemäß Anspruch 1 und 2, dadurch gekennzeichnet, daß der eingesetzte Edelmetallträgerkatalysator zwischen 0,1 und 10 Gew.-% Edelmetall enthält, das zwischen 100 und 30 % aus Rh besteht.

4. Verfahren gemäß Anspruch 1 bis 3, dadurch gekennzeichnet, daß der Edelmetallträgerkatalysator zwischen 0,05 und 5 Gew.-% Cr, Mo, W, Mn und/oder Re, weiterhin zwischen 0,05 und 15 Gew.-% Alkali- oder Erdalkalimetallionen und gegebenenfalls zwischen 0,05 und 3 Gew.-% Schwefel in Form von Verbindungen enthält.

5. Verfahren gemäß der Ansprüche 1 bis 4 zur katalytischen Hydrierung von Anilin, C₁-C₆-Alkylanilinen sowohl im Kern wie am Stickstoff alkyliert, C₁-C₆-alkylierte Diaminobenzolen, Aminonaphthalinen und C₁-C₃-alkylierten Aminonaphthalinen, Diaminonaphthalinen und Diamino-diphenyl-C₁-C₃-alkanen.

6. Verfahren zur Herstellung eines Edelmetallträgerkatalysators gekennzeichnet durch
i) Aufbringen von Metallsalzen oder Oxiden der Reihe Cr, Mo, W, Mn und/oder Re oder Gemischen davon durch gemeinsames Ausfällen von Oxid-Hydroxid-Gemischen auf dem getränkten Träger mit Alkali-, Erdalkali- oder Ammonium-Hydroxiden und gegebenenfalls Auswaschen löslicher Anteile mit Wasser,
ii) Trocknen und Erhitzen dieses Trägers auf 200 bis 450°C,
iii) Aufbringen von Rh und gegebenenfalls eines Edelmetalls der Reihe Ir, Ru, Os, Pd, Pt und/oder Alkali- oder Erdalkali-Hydroxid und gegebenenfalls Alkali- oder Erdalkalisulfat,
iv) Trocknen und Reduzieren mit Wasserstoff bei Temperaturen zwischen 80 und 350°C.

7. Edelmetallträgerkatalysator hergestellt nach Anspruch 6.

8. Verwendung des Edelmetallträgerkatalysators gemäß Anspruch 7 bei der katalytischen Niederdruckhydrierung von aromatischen Aminen zu cycloaliphatischen Aminen.

9. Edelmetallträgerkatalysator gemäß Anspruch 7, dadurch gekennzeichnet, daß der Träger aus Tonerden, Al₂O₃ in den verschiedenen Modifikationen (α, κ, η, γ), für Edelmetalle übliche Träger wie TiO₂, Kieselgur, Silicagel, BaCO₃, CaCO₃, ZnO, MgO, Bims, ZrO₂, Aktivkohle oder den Oxiden bzw. Oxidhydraten von Cr, Mo, W, Mn und/oder Re besteht.
